# EUROPEAN PATENT SPECIFICATION

(11) **EP 2 956 081 B1**
(45) Date of publication and mention of the grant of the patent: **10.10.2018**
(21) Application number: 14751629.8
(22) Date of filing: 05.02.2014
(51) Int. Cl.: A61B 46/20, A61B 46/27, A61B 17/3203, A61M 1/00, A61M 3/02

(54) **MEDICAL IRRIGATION DEVICE AND METHOD**
MEDIZINISCHE SPÜLVORRICHTUNG UND VERFAHREN
DISPOSITIF D'IRRIGATION MÉDICALE ET PROCÉDÉ

(30) Priority: 13.02.2013 US 201313766063
(43) Date of publication of application: 23.12.2015
(73) Proprietor: Bone Foam Inc., Plymouth, Minnesota 55447 (US); Parsell, Doug, Rigeland, Mississippi 39157 (US); Robran, Chad, Plymouth, Minnesota 55447 (US)
(72) Inventor: PARSELL, Doug, Ridgeland, Mississippi 39157 (US); ROBRAN, Chad, Plymouth, Minnesota 55447 (US)
(74) Representative: Schwarz & Partner Patentanwälte OG
(86) International application number: PCT/US2014/014913
(87) International publication number: WO 2014/126764

(56) References cited:
- DE-U1-202011 109 057
- US-A- 5 312 385
- US-A- 5 609 163
- US-A- 5 609 163
- US-A1- 2004 225 265
- US-A1- 2004 225 265
- US-B1- 6 402 724

## Description

### BACKGROUND OF THE INVENTION

### 1. The Field of the Invention

The present invention relates to devices and methods for use in removing biological debris from patient extremities. More specifically, the present invention relates to medical irrigation devices and methods for containing the fluid and solid biological debris ejected as a result of irrigation and/or debridement using a fluid.

### 2. Background and Relevant Art

Within the medical arts, it is common practice to remove infected and/or necrotic tissues to allow for increased healing rates and to decrease infection risk. The clinical procedure for the above mentioned action is termed wound debridement. A common component of the debridement process is copious fluid irrigation of the targeted tissue areas. The fluids used are typically sterile saline but fluid with additional antimicrobial agents may also be utilized.

In general, known techniques for tissue debridement include surgical, chemical, mechanical, and autolytic. Surgical debridement techniques include conventional surgical techniques, involving the use of sharp medical instruments, and hydrosurgical techniques, involving the use of a high-pressure stream or spray of jetted fluid.

In hydrosurgical debridement, infected and/or necrotic tissue (*i.e.,* tissue that is dead, burned, diseased, infected, etc.), is ablated using a stream or spray of water, saline, or other fluid directed under very high pressure at the tissue to be removed. Because large amounts of fluid are required, and because the high pressure of the fluid against the targeted necrotic tissue can result in uncontrolled emission of fluid as well as the ablated pieces of infected and/or necrotic tissue, hydrosurgical debridement can be unsanitary and messy.

Uncontrolled tissue and fluid emission associated with hydrosurgical debridement can create problems for medical staff, such as contamination of equipment, clothing, face shields or eye protection. In addition, hydrosurgical debridement procedures can require the complete turnover and re-sterilization of some or all of equipment in the operating room, as well as a thorough disinfection and sterilization of every surface in the operating room. Turnover, sterilization and disinfection procedures are labor-intensive, time-consuming, and expensive. As such, there exists a need for extremity supporting structures that facilitate the effective and convenient deployment of the irrigation and debridement process.
Document US 2004/225265 A1 discloses a wound irrigation kit including a basin for collecting irrigation fluid. The kit includes a grommet that can be readily incorporated into the basin to allow for active draining of the basin during the irrigation procedure. The kit also includes a flexible irrigation shield that can be attached to an existing irrigation device and that can be readily modified to vary its length. The irrigation shield may be disposed over the region of the patient to be treated.

Document US 5 312 385 A discloses an apparatus and a method for performing protected pulse irrigation.

Document DE 20 2011 109057 U1 discloses an apparatus for draining and sucking liquids from wounds.

Document US 5 609 163 A discloses a metal basin including a grid of metal rods for supporting a limb extremity above irrigation fluids collected in the bottom of the basin.

One of the major disadvantages of current irrigation devices consists in the lack of an effective support of the patient's extremity.

The objective of the present invention is therefore to provide a medical irrigation device and method that provides an effective support of the patient's extremity, while simultaneously providing effective channeling of debris and liquid to the drainage passageway.

This objective is achieved by an apparatus according to the characterizing portion of claim 1.

### BRIEF SUMMARY

Disclosed herein are embodiments of an apparatus for supporting an extremity during tissue irrigation and/or debridement and capturing fluid and tissue resulting from tissue irrigation and/or debridement, and methods of irrigating and/or debriding tissue of an extremity and capturing fluid and tissue resulting therefrom. The apparatus and methods can provide improved sanitary conditions for a patient and personnel involved in a tissue irrigation and/or debridement procedure, as well as a cleaner environment in the operating room.

According to one embodiment, an apparatus for supporting an extremity during tissue irrigation and/or debridement and capturing fluid and/or tissue, according to independent claim 1, includes an extremity-supporting base configured to support an extremity of a patient during tissue irrigation and/or debridement. The base has a proximal end positioned nearest a patient's body during use and a distal end opposite the proximal end. A barrier shroud that is positionable so as to at least partially enclose an extremity placed on the extremity-supporting base. A drainage passageway in fluid communication with the extremity-supporting base and barrier shroud, which provides controlled drainage of fluid and tissue from the extremity-supporting base and barrier shroud during tissue irrigation and/or debridement. The drainage passageway can be positioned at or near the distal end of the base. A dam at the distal end of the base or associated with the barrier shroud can assist is directing fluids toward the drainage passageway.

The extremity-supporting base includes a trough, such as a concave trough, for cradling an extremity. The trough may have a trough bottom and a side wall on either side of the trough bottom. The extremity-supporting base may be downwardly angled toward the drainage passageway to facilitate movement of fluid and tissue toward the drainage passageway during tissue irrigation and/or debridement. According to one embodiment, the drainage passageway is positioned at or near the distal end of the base to facilitate movement of fluid and tissue away from the patient's body during tissue irrigation and/or debridement. The drainage passageway can be provided by a hole through the base and/or barrier shroud. A drainage tube may be attached to the drainage passageway to facilitate drainage of fluid and tissue into a receiving vessel. Drainage may be gravitational or aspiration assisted. The base can be configured to permit a portion of an extremity to extend beyond the distal end of the base (*e.g.,* by shortening or eliminating the dam at the distal end of the base).

According to one embodiment, the barrier shroud comprises a flexible sheet material, such as a water-proof polymer. One or more braces that cooperate with the flexible barrier shroud may advantageously maintain the barrier shroud in a desired elevated configuration above the extremity-supporting base and relative to an extremity during tissue irrigation and/or debridement (*e.g.*, to provide space between the shroud and extremity). Alternatively, the shroud can be extended between the outer vertical walls defining the trough, such as when the trough is sufficiently deep to contain most or all of the extremity being treated. The barrier shroud may include one or more fasteners that permit selective opening and closing of the barrier shroud relative to an extremity placed on the extremity-supporting base. The barrier shroud may include an opening in an upper region that permits access to the extremity by an irrigation and/or debridement instrument, such as a high pressure irrigation device.

According to one embodiment, the extremity-supporting base may be formed from an open-cell foam material (*e.g*., flexible polyurethane foam). A flexible, fluid-impermeable coating may be positioned over at least a portion of the open-cell foam material to provide additional sterility and ease of cleaning.

According to an embodiment, the extremity-supporting base includes a dam at the distal end of the extremity-supporting base that assists in drainage of fluids through the drainage passageway. The dam may be sufficiently short and/or have a concave curvature so that a portion of a patient extremity can comfortably extend beyond the distal end of the extremity-supporting base. The barrier shroud may be positioned such that it substantially covers both the extremity-supporting base and the patient extremity resting in or on the extremity-supporting base.

A drainage passageway can be provided at or near the proximal side of the dam, preferably at the deepest portion of the trough of the extremity-supporting base. The drainage passageway may comprise a tray- or cup-like receiving structure formed of a rigid or semi-rigid material for collecting fluid and debris produced during cleaning of a patient extremity. The receiving structure includes an outlet positioned therein, preferably in a bottom or side of the receiving structure. A drainage tube may be attached to the receiving structure outlet. Gravity causes fluid and tissue to collect on the proximal side of the dam. Gravity and/or aspiration further cause the collected fluid to travel into the receiving structure and out of the receiving structure into the drainage tube, through the receiving structure outlet. Tissue and fluid collected at the base of the dam may be urged by gravity or aspiration to the opening for the receiving structure, which may be covered by a grate or other filtering structure designed to capture larger tissue and other solid debris that might clog the drainage tube but permit fluid and smaller debris or tissue particles to pass through and into the receiving structure.

A ramp for supporting the patient extremity may be positioned in the trough of the extremity-supporting base near the distal end, such as proximal to the receiving structure opening. The ramp may or may not be attached to the receiving structure or to the grate that covers the receiving structure opening. The ramp may be a separate structure or may be formed into the trough of the extremity-supporting base itself. The ramp can be configured such a distal end of the ramp is taller than a proximal end relative to the base. In this way, the ramp advantageously positions the patient extremity so as to not block the receiving structure. In the case where a portion of the patient extremity extends beyond the distal end of the base, the ramp can help position that portion of the extremity so to raise it above or permit it to rest comfortably on the dam so as to prevent deformation or collapse of the dam. In this way, the integrity of the dam may be maintained so that tissue and fluid collected at the base of the dam do not escape the extremity-supporting base.

According to another embodiment, a method of capturing fluid and tissue produced while of irrigating and/or debriding tissue of an extremity according to independent claim 14, comprises providing an apparatus as defined in claim 1, and collecting fluid and/or tissue resulting from tissue irrigation and /or debridement.

The method may include gravitational and/or aspiration assisted direction of fluid and tissue to a receiving structure positioned within a drainage passageway of the base, which structure may or may not be covered by a grate that captures the solid debris and allows drainage of fluid. The method may further include gravitational and/or aspiration assisted drainage, through a drainage tube in fluid communication with the receiving structure, of the fluid directed into the receiving structure.

This Brief Summary is provided to introduce in a simplified form a selection of concepts that are further described below in the Detailed Description. This Brief Summary is not intended to identify key or essential features of the claimed subject matter, nor is it intended to limit the scope of the claimed subject matter. Additional features and advantages of the invention will be set forth in the description which follows, and in part will be evident to persons of ordinary skill in the art from the description and appended claims, or may be learned by such persons through the practice of the invention.

### BRIEF DESCRIPTION OF THE DRAWINGS

In order to describe the manner in which the above-recited and other advantages and features of the invention can be obtained, a more particular description of the invention briefly described above will be rendered by reference to specific embodiments thereof which are illustrated in the appended drawings. It should be noted that the figures are not necessarily drawn to scale, and that elements of similar structure or function are generally represented by like reference numerals for illustrative purposes throughout the figures. These drawings depict only certain embodiments of the invention and are not therefore to be considered to be limiting of its scope.
Figure 1 is an example embodiment of an extremity-supporting base having a downward sloping concave trough with a dam and drainage opening at a distal end.
Figure 2 is an alternative embodiment of an extremity-supporting base having a downward sloping concave trough and being configured so as to permit an extremity to extend beyond the distal end.
Figure 3 illustrates an example embodiment of an apparatus for supporting an extremity during tissue irrigation and/or debridement and capturing fluid and/or tissue, which includes a base supporting an extremity (*e.g*., leg, with a portion of the foot shown extending beyond the distal end of the base) and a flexible barrier shroud enclosing a distal portion of the extremity.
Figure 4 illustrates another embodiment of an apparatus for supporting an extremity during tissue irrigation and/or debridement and capturing fluid and/or tissue, which includes an extremity-supporting base and a flexible barrier shroud with fasteners that permit selective fastening and unfastening of the shroud during placement and removal of an extremity on the base.
Figure 5 illustrates the apparatus of Figure 4 supporting an extremity ready to undergo tissue irrigation and/or debridement and capture fluid and/or tissue by a drainage tube, with a portion of the extremity shown extending beyond the distal end of the base.
Figure 6 is an alternative view of the embodiment illustrated in Figure 5.
Figure 7 illustrates a modified apparatus for supporting an extremity during tissue irrigation and/or debridement and capturing fluid and/or tissue, in which the shroud includes an opening in an upper portion to facilitate access by an irrigation and/or debridement instrument to the patient extremity.
Figure 8 illustrates an embodiment of an apparatus for supporting an extremity during tissue irrigation and/or debridement including an extremity-supporting base, a shortened concave dam at the distal end, a drainage passageway near the dam, a receiving structure within the drainage passageway, a drainage tube in communication with the receiving structure, a grate covering the receiving structure, and a ramp configured to elevate the patient extremity.

### DETAILED DESCRIPTION

The following are example embodiments of an apparatus for supporting an extremity during tissue irrigation and/or debridement and capturing fluid and tissue resulting therefrom tissue, and methods of irrigating and/or debriding tissue of an extremity and capturing fluid and/or tissue resulting therefrom. Irrigation and/or debridement can be performed using any desired lavage apparatus. By way of example, lavage could be performed by jet-lavage, syringe lavage, manual squeeze bulb, saline or other fluid bag lavage, or simply pouring a fluid over a wound.

According to one embodiment, an extremity supporting medical irrigation ramp (or apparatus for supporting an extremity during tissue irrigation and/or debridement and capturing fluid and/or tissue resulting therefrom) is composed of two primary elements: an extremity-supporting, fluid-channeling element (or extremity-supporting base) and a sterile, barrier element (or barrier shroud). In one embodiment, the extremity-supporting base may be constructed from open cell foam material at least partially covered with a fluid impermeable, flexible exterior coating. The exterior coating is may comprise a polymeric, non-latex composition.

The extremity-supporting base may include a concave surface that supports the patient's extremity (*e.g.*, arm or leg). The concavity of the extremity-supporting element can function to channel fluids applied during the irrigation and/or debridement procedure, and also tissue removed during irrigation and/or debridement, toward a distal end of the device. The extremity-supporting base may further include a downward slope, which slopes downward toward a distal end of the extremity-supporting base. In one embodiment, a dam feature at a distal end of the apparatus allows for pooling of applied fluids and removed tissues. A drainage passageway, preferably in the form of a drainage hole, at or near the distal end of the base, allows for the continuous removal/evacuation of fluids and tissue. The dam might form part of the extremity-supporting base and/or the barrier shroud. The base may be configured to permit a portion of the extremity to extend beyond the distal end (*e.g.,* by shortening or eliminating the dam).

For each individual irrigation and/or debridement case (*i.e.,* each new patient extremity), a new sterile, barrier element or shroud may be used by placing between the patient's extremity and the extremity-supporting base. In this way, the extremity-supporting base can be kept sterile and re-used if desirable while the barrier shroud is a single-use, disposable feature. Alternatively, the base may itself be disposable, in which case the shroud may be positioned to substantially cover both the extremity and the extremity-supporting base. The barrier shroud can be a smooth plastic sheet that easily conforms to the concave shape of the extremity-supporting base and is advantageously of adequate size to cover all potential patient extremity- contacting surfaces of the extremity-supporting base.

According to one embodiment, the barrier element may include a drainage hole and an integrated, semi-rigid drainage tube that is of a diameter to slide into a drainage hole at the distal extent of the extremity-supporting base (when included). The drainage tube can be of sufficient length to extend significantly beyond the bottom surface of the extremity-supporting base, when fully engaged. Alternatively, a drainage tube can be attached to the drainage hole of the barrier element when positioned beyond a distal end of the extremity-supporting element. The drainage tube allows for fluids applied during the irrigation and/or debridement procedure to exit the device via gravity-assisted, fluid movement into a receiving vessel or, alternatively, via suction assisted evacuation of fluids.

To further capture fluid droplets that may possess an upwards trajectory during the irrigation and/or debridement procedure, the sterile covering element or shroud may comprise a "green house" structure (*e.g.,* by means of hoops or braces that hold the barrier shroud in a desired configuration during use). This feature can attach at the lateral edge of the device and arch over the extremity. The "green house" structure can be made from clear plastic to allow for adequate visualization of the irrigation and/or debridement field. Right and left side slits may run along the length of the "green house" structure, so as to allow for insertion of a tip of an irrigation device through the plastic barrier and direct access to the irrigation and/or debridement site.

The top portion of the barrier shroud may include an opening formed therein to allow a hydro surgical irrigation and/or debridement device to access the irrigation and/or debridement site of the extremity. The top portion and bottom portion may be joined together around the edges in either a permanent manner or in a manner designed to allow the shroud to be opened and closed (*e.g.,* like a sleeping bag). The top portion and bottom portion of the shroud may include fasteners such as snaps, Velcro® (hook and loop), zippers, hooks, clasps, or other fastening elements known in the art which can allow the shroud to be selectively opened and closed.

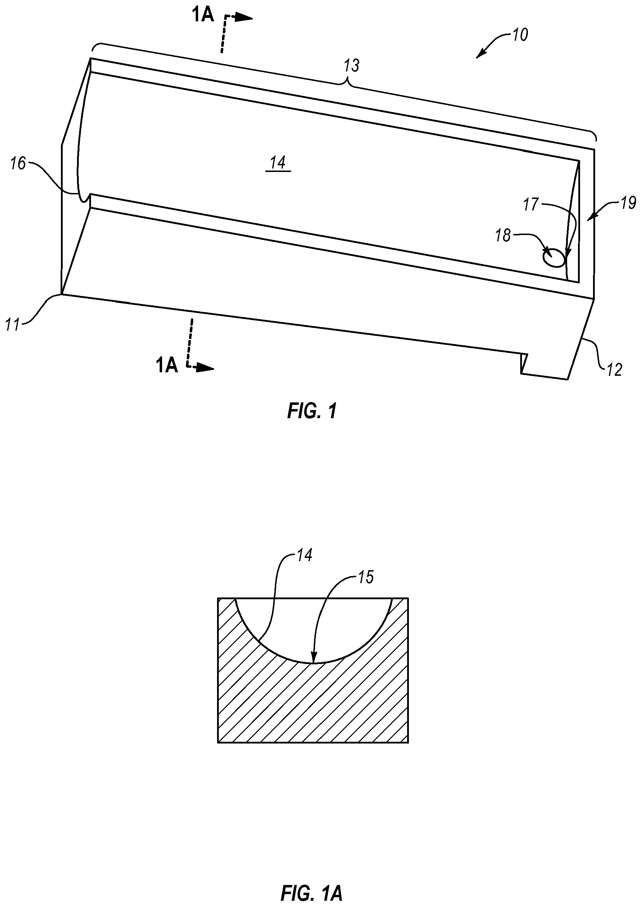

Referring now to Figure 1, an embodiment of an extremity-supporting base 10 is shown. Base 10 has a proximal end 11 and a distal end 12 defining a length 13 between proximal end 11 and distal end 12. Proximal end 11 is typically positioned nearest to the patient's body (*e.g.,* torso in the case of a leg or shoulder in the case of an arm). Distal end 12 is typically the end farthest away from the patient's body.

Base 10 is configured for use as a support for a patient extremity, such as an arm or a leg, which can be further enclosed within a shroud, discussed below, during the process of hydrosurgical wound irrigation and/or debridement. Base has a concave support surface to help cradle and hold a patient extremity in a desired orientation, and which can direct fluid and tissue debris associated with hydrosurgical irrigation and/or debridement toward the lowest point of a downwardly sloping support surface. Base 10 may therefore be constructed such that a trough 14 having a concave shape extends along part or all of the length 13 of base 10. Trough 14 as illustrated includes a trough bottom 15 that also runs along part or all of the length 13 of base 10. Trough 14 and trough bottom 15 are more particularly illustrated in Figure 1A, which is a cross-sectional view of base 10 as seen from proximal end 11.

To control runoff of fluid and tissue debris associated with an irrigation and/or debridement procedure, trough 14 can be sloped downward along the base length 13 from a high point 16 at proximal end 11 to low point 17 at distal end 12. In the illustrated embodiment, positioned at or near the low point 17 is a base drainage opening or passageway 18. A base dam 19 is positioned at distal end 12 in close proximity to base drainage opening 18 such that excess fluid and/or tissue debris associated with irrigation and/or debridement can build-up in the area adjacent to base drainage opening 18, which facilitates controlled drainage of such fluid and tissue debris through passageway or hole 18. Drainage can be by gravity alone or assisted by applied vacuum suction. In this embodiment, dam 19 extends upward to the same height as the sidewalls defining the trough 14 to prevent overflow of fluids beyond the distal end of trough 14 over dam 19.

In an alternative embodiment (not shown), the trough bottom may include more than one high point on either side of a low point. The multiple high points and trough bottom low point may be arranged such that a trough bottom low point is located at or near the center of the base to promote drainage through a passageway at this location. Alternatively, the trough bottom may include a high point in the center and low points at the proximal and distal ends.

Base 10 may be made of open-cell foam and may have a coating thereof that is fluid-impermeable and flexible. The coating on base 10 may be formed of a polymeric, non-latex composition.

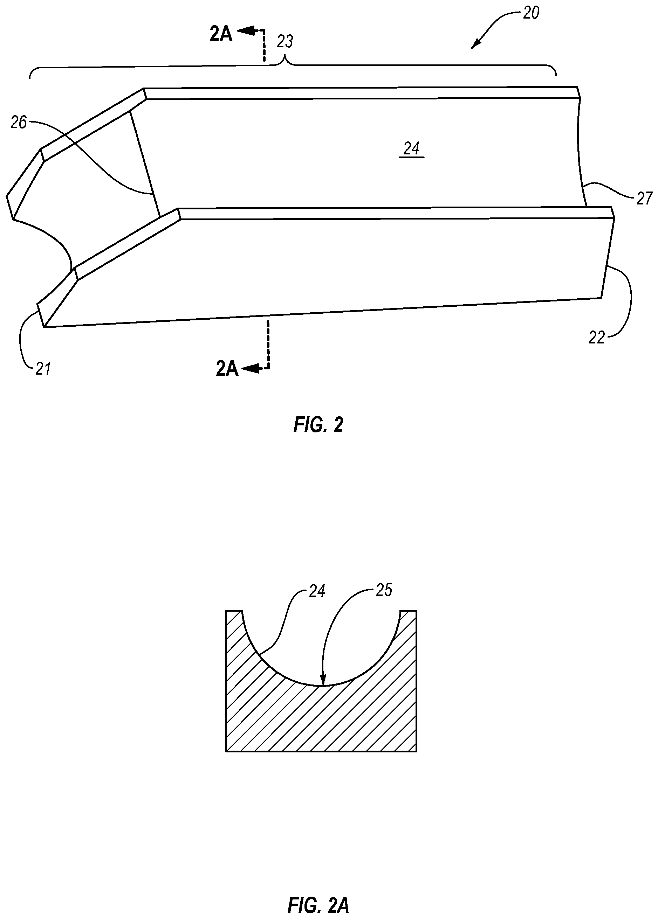

Referring now to Figure 2, another embodiment of an extremity-supporting base 20 is illustrated. Some features of base 20 are the same as those of base 10 with two primary exceptions: base 20 has neither a base dam nor a base drainage opening (such as elements 18 and 19, respectively, shown in Figure 1). Otherwise, base 20 includes a proximal end 21, a distal end 22, a length 23, a trough 24, a trough bottom 25 (shown in Figure 2A, which is a cross-sectional view of base 20 viewed from distal end 22), a trough bottom high point 26, and a trough bottom low point 27. Thus, trough 24 slopes downwardly toward distal end 22.

Like base 10, base 20 is designed for use as a support for a patient extremity, such as an arm or a leg, during the process of tissue irrigation and/or debridement. Because base 20 does not have a base dam or a base drainage opening, an embodiment of a barrier shroud (discussed below) that cooperates with base 20 may be different than the shroud embodiment designed to cooperate with base 10. Specifically, the shroud drainage opening does not align with a base drainage opening when using base 20 because base 20 has no base drainage opening. In one embodiment, base 20 may be used with and support a barrier shroud having a shroud dam and a shroud drainage opening that are positioned beyond distal end 22 of base 20, as will be discussed in more detail below.

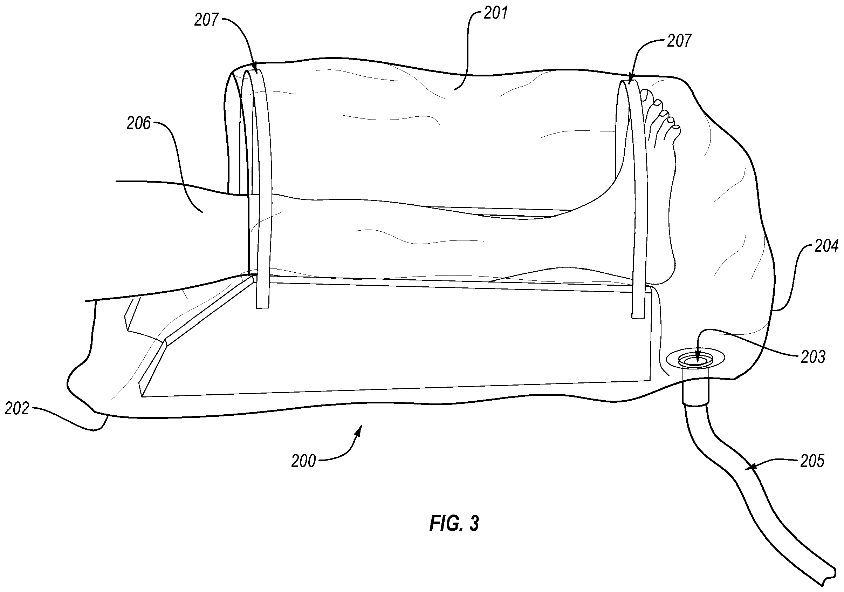

Like base 10, base 20 may be made of open-cell foam and may have a coating that is fluid-impermeable and flexible. The coating on base 20 may be formed of a polymeric, non-latex composition. However, unlike the embodiment shown in Figure 1, the embodiment illustrated in Figure 2 permits a portion of an extremity resting on the base to comfortably extend beyond the distal end (*e.g.,* as illustrated in Figures 3 and 5-7) (*i.e.,* because it is not blocked by the tall dam shown in the Figure 1).

Although not depicted in the drawings, base 10 may operate together with a shroud that has a shroud drainage opening and a drainage tube that cooperate with base drainage opening 18 of base 10 such that the shroud includes a drainage opening which substantially lines up over base drainage opening 18 and a drainage tube that fits down and through base drainage opening 18 and extends beyond an underside of base 10.

Figure 3 illustrates an extremity-supporting base similar to base 20 in cooperation with an embodiment of a barrier shroud 200. This embodiment of shroud 200 as shown has a top portion 201, a bottom portion 202, and a shroud drainage opening 203. A shroud dam 204 is formed by a raised or vertical portion just beyond shroud drainage opening 203 interconnecting top portion 201 and bottom portion 202. Shroud drainage opening 203 is attached to a drainage tube 205 that extends down and leads away from shroud 200. Shroud dam 204 is positioned beyond and below a distal end of the base in close proximity to shroud drainage opening 203 such that the excess fluid and/or tissue debris associated with tissue irrigation and/or debridement may pool in an area of shroud 200 beyond and below the distal end of the base. Fluid and tissue may exit shroud drainage opening 203 with the aid of gravity and/or applied vacuum suction.

The availability of vacuum suction may alternatively permit shroud drainage opening 203 to be alternatively located in other regions of the shroud 200, such as in dam 24 or even top portion 201 of shroud 200 if top and bottom portions 201, 202 of shroud 200 are sealed at all points except the opening adjacent to the proximal end of the base.

One purpose of barrier shroud 200 is to contain fluid and removed tissue that may tend to spatter or be emitted from patient extremity 206 during the irrigation and/or debridement process. Consistent with this purpose, the material for shroud 200 is advantageously fluid-impermeable or fluid-resistant. However, since shroud 200 is intended only for a single use and need not endure repeated exposure to fluid, materials that are not strictly fluid-resistant or fluid-impermeable may suffice.

As used in one particular embodiment, shroud 200 is positioned over patient extremity 206 such that bottom portion 202 of shroud 200 lies beneath extremity 206 but above the base, which supports the shroud-encased patient extremity 206. Top portion 201 of shroud 200 further includes one or a plurality of braces 207 operatively associated with top portion 201, although two braces 207 are shown in this illustrated embodiment. One purpose of brace 207 is to suspend top portion 201 over patient extremity 206 such that top portion 201 does not come into substantial contact with patient extremity 206. Another purpose of brace 207 is to provide the surgeon and medical staff with sufficient working space above patient extremity 206 but below top portion 201 so as to allow for the effective carrying out of a hydrosurgical irrigation and/or debridement procedure.

The material used for brace 207 and the mechanism for operatively relating brace 207 with top portion 201 may be selected from any of a number of materials and methods consistent with accomplishing these purposes. Brace 207 may be operatively associated with top portion 201 in any of a number of conventional ways, including, but not limited to, being slidably positioned in a slot formed in top portion 201, fixedly attached to top portion 201 with adhesive, or integrally formed into the material of top portion 201 during manufacture. Brace 207 may be of fixed shape, such as the arcuate shape illustrated in Figure 3, or may be of a deformable material, such as a soft metal, that can be manipulated into a desired shape.

In the case where the sidewalls of the trough are sufficiently high and trough sufficiently deep to contain most or all of an extremity, it may be possible to eliminate brace 207 and simply stretch or extend the shroud between the sidewalls defining the trough in order to enclose at least a portion of the extremity being treated.

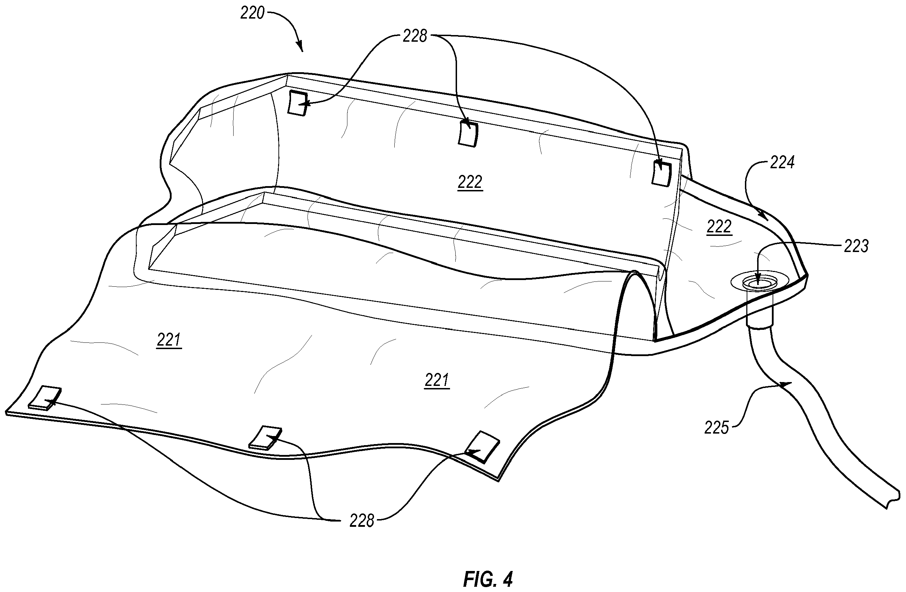

Shroud 200 of Figure 3 may be formed in a manner similar to that shown in Figure 4 such that top portion 201 opens like a flap, or the top part of a sleeping bag, wherein top portion 201 is permanently joined to bottom portion 202 along one edge of shroud 200 (adjacent the length 23 of the base 20) and is attachable to bottom portion 202 along the opposite edge using known fastening devices such as snaps, Velcro® (hook and loop), buttons, clasps, hooks or zippers.

Top portion 201 and bottom portion 202 of shroud 200 may be joined in the manner shown in Figure 3, that is, permanently joined along both opposing side edges and may or may not be permanently joined along the bottom edge, adjacent distal end of the base 20.

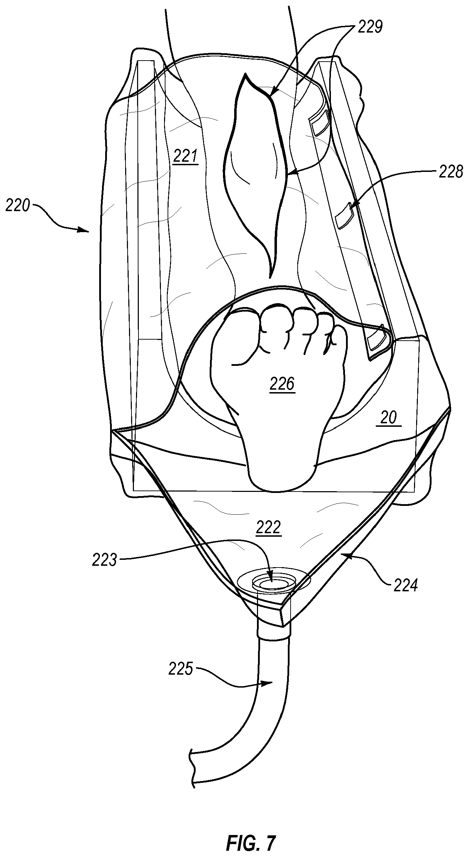

Shroud 200 may also include one or more slit openings, such as the opening 229 shown in Figure 7, formed in top portion 201 so as to allow access to patient extremity 206 by an irrigation and/or debridement instrument during an irrigation and/or debridement procedure.

Referring now to Figure 4, another embodiment of an illustrative shroud 220 is shown. The features of shroud 220 are similar to those of shroud 200 shown in Figure 3, with three primary exceptions: shroud 220 has no brace 207; shroud 220 is open at both ends, not just at the end associated with the proximal end of the base; and shroud 220 has a separate shroud dam 224 formed into or attached to a bottom portion 222 and does not, as is illustrated in Figure 3, rely upon the region where the top and bottom portions meet to function as the shroud dam. Otherwise, shroud 220 has a top portion 221, a bottom portion 222, a shroud drainage opening 223, a shroud dam 224, and a drainage tube 225.

Shroud dam 224 can be a ridge-like structure formed into bottom portion 222 of shroud 220 and may be positioned beyond and below the distal end of the base in close proximity to shroud drainage opening 223 such that fluid and/or tissue debris associated with irrigation and/or debridement may pool in the area near shroud drainage opening 223 and exit shroud drainage opening 223 with the aid of either gravity or applied vacuum suction. Alternatively, shroud dam 224 may be a separately formed structure that is fixedly attached to shroud bottom 222 using known methods, such as heat to the shroud dam 224 to shroud bottom portion 222, or glue, or other adhesives.

As can be seen in Figure 4, top portion 221 can open and close like a flap, and shroud 220 is open at both ends adjacent the proximal and distal ends of the base. Top portion 221 is permanently joined to bottom portion 222 along one edge of shroud 220 and is releasably attachable to bottom portion 222 along the opposite edge using known fastening devices 228 such as snaps, Velcro®, buttons, hooks, clasps or zippers.

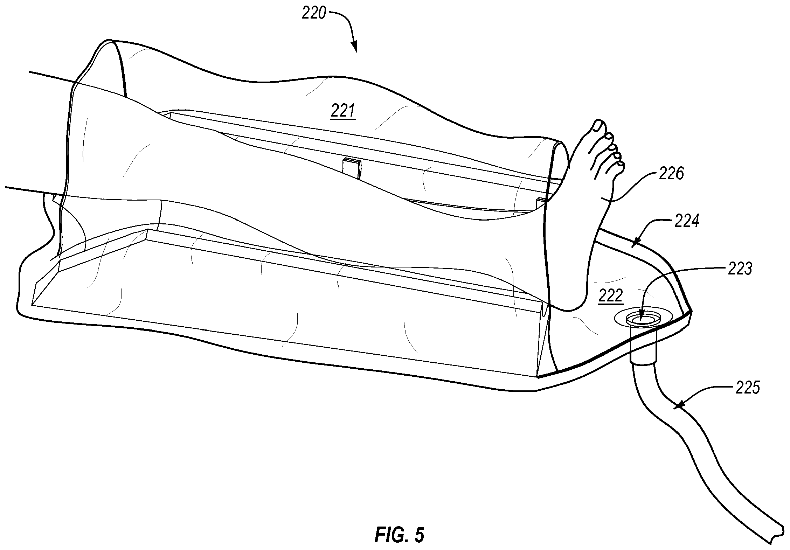

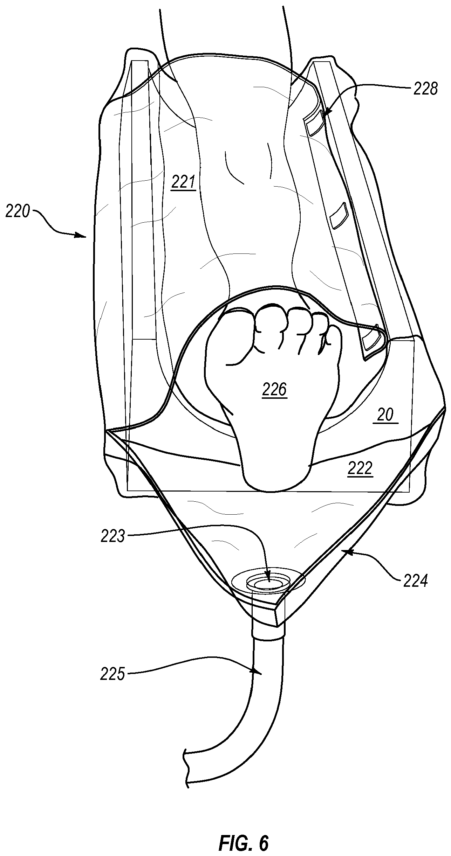

Figures 5-7 show the embodiment of Figure 4 in use, where the base and shroud 220 cooperate to support and enclose a patient extremity 226. Figure 6 shows the same configuration of base 20 and shroud 220, but viewed from the distal end 22 of the base 20 towards the proximal end 21 of the base 20. Figure 7 is the same as Figure 6 but illustrates the incorporation of at least one opening 229, in the form of a slit in the embodiment shown, in top portion 221 of shroud 220 so as to allow access to patient extremity 226 by the irrigation and/or debridement instrument being operated by the medical personnel.

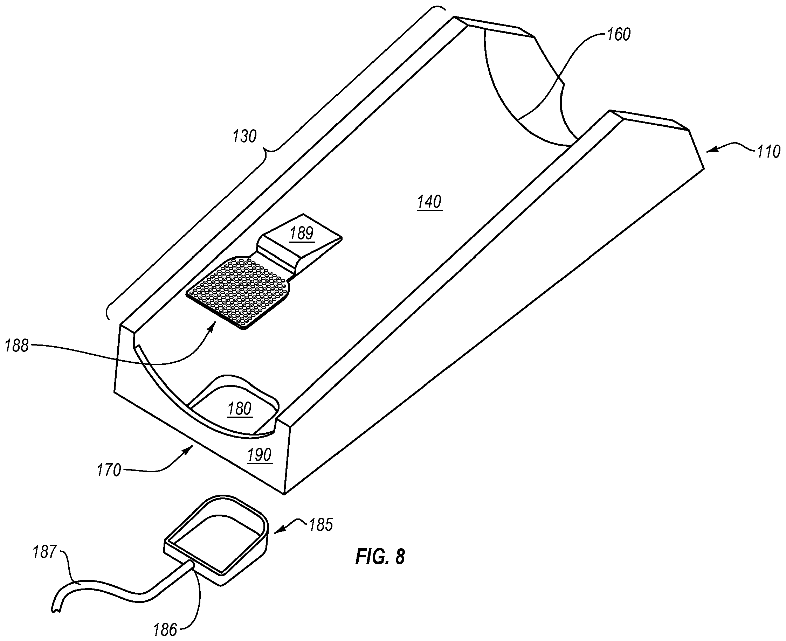

Figure 8 shows an embodiment of an extremity-supporting base that is a modification of the embodiment shown in Figure 1. The extremity-supporting base includes a trough 140 can be sloped downward along the base length 130 from a high point 160 at proximal end 110 to low point 170 at distal end 120. A shortened (*e.g.*, concave) base dam 190 is provided at the distal end 120 of the extremity-supporting base, the base dam 190 positioned to enclose the distal end 120 of the extremity-supporting base. A barrier shroud (not shown) may be positioned such that it substantially covers both the extremity-supporting base and the patient extremity (not shown) resting in or on the extremity-supporting base. A drainage passageway 180 is provided at or near the point where base dam 190 meets low point 170 of trough 140, preferably at the deepest portion of trough 140 of the extremity-supporting base.

Drainage passageway 180 includes a tray- or cup-like receiving structure 185 formed of a rigid or semi-rigid material, and having a receiving structure outlet 186 positioned therein, preferably in a bottom or side of receiving structure 185. A drainage tube 187 may be attached to receiving structure outlet 186. Gravity causes fluid and tissue to collect at the intersection of trough 140 and base dam 190. Gravity and/or aspiration further cause the collected fluid to travel into receiving structure 185 and out of the receiving structure into drainage tube 187, through the receiving structure outlet 186. Tissue collected at the foot of base dam 190 may be urged by gravity or aspiration to the opening for the receiving structure, which may be covered by a grate 188 or other filtering structure designed to capture tissue and other solid debris, but to allow fluid to pass through and into the receiving structure 185.

A ramp 189 for supporting a portion of a patient extremity may be positioned in trough 140 of the extremity-supporting base near distal end 120, preferably near to the receiving structure opening positioned in drainage passageway 180. Ramp 189 may or may not be attached to receiving structure 185 or to grate 188 covering the receiving structure opening positioned in drainage passageway 180. Ramp 189 may be a separate structure or may be formed into trough 140 of the extremity-supporting base itself. Ramp 189 is positioned such that it is taller at a distal end than a proximal end. Ramp 189 advantageously elevates the patient extremity (not shown) to a degree sufficient so that a portion of the patient extremity that may extend beyond the distal end 120 of the extremity-supporting base is positioned above or comfortably rests upon base dam (*e.g.,* to not cause deformation or collapse of base dam 190). In this way, the integrity of base dam 190 may be maintained so that tissue and fluid collected at the foot of base dam 190 do not escape the extremity-supporting base as a result of the patient extremity causing a deformation or collapse of base dam 190.

## Claims

1. An apparatus for supporting an extremity during tissue irrigation and/or debridement and capturing fluid and tissue resulting therefrom, comprising:
an extremity-supporting base (10, 20) configured to support an extremity of a patient during tissue irrigation and/or debridement, said base (10, 20) having a proximal end (11, 21, 110) positioned nearest a patient's body during use and a distal end (12, 22, 120) opposite said proximal end (11, 21, 110), the base (10, 20) being configured to permit a portion of an extremity to extend beyond the distal end (11, 21, 110), wherein said extremity-supporting base (10, 20) forming a trough (14, 24, 140) for cradling an extremity on said extremity-supporting base (10, 20), said trough (14, 24, 140) extending along the length of the of the extremity-supporting base (10, 20) from a point at or near the proximal end (11, 21, 110) of the extremity-supporting base (10, 20) to a point at or near the distal end (12, 22, 120) of the extremity-supporting base (10, 20), said trough (14, 24, 140) being unenclosed at least toward the proximal end (11, 21, 110) of the extremity-supporting base (10, 20);
a barrier shroud (200, 220) that is positionable so as to at least partially enclose an extremity placed on said extremity-supporting base (10, 20); and
a drainage passageway (18, 180, 203, 223) in fluid communication with said extremity-supporting base (10, 20) and said barrier shroud (200, 220) that provides controlled drainage of fluid and tissue from said extremity-supporting base (10, 20) and said barrier shroud (200, 220) during tissue irrigation and/or debridement.

2. An apparatus as in claim 1, wherein said trough (14, 24, 140) includes a trough bottom (15, 25) and a side wall on either side of said trough bottom (15, 25).

3. An apparatus as in claim 1 or 2, wherein said extremity-supporting base (10, 20) is downwardly angled toward said drainage passageway (18, 180, 203, 223) to facilitate movement of fluid and tissue toward said drainage passageway (18, 180, 203, 223) during tissue irrigation and/or debridement.

4. An apparatus as in claim 3, wherein said drainage passageway (18, 180, 203, 223) is positioned at or near said distal end (12, 22, 120) of said extremity-supporting base (10, 20) to
facilitate movement of fluid and tissue away from the patient's body during tissue irrigation and/or debridement.

5. An apparatus as in claim 3 or 4, wherein said drainage passageway comprises a drainage hole (18, 180) through said extremity-supporting base (10) at or near a low point of said downwardly angled base (10).

6. An apparatus as in claim 3 or claim 4, wherein said extremity-supporting base (20) is configured to channel fluids and/or tissue toward and beyond the distal end (22) of the extremity-supporting base (20), wherein said drainage passageway comprises a drainage hole (203, 223) formed in said barrier shroud (200, 220), the drainage hole (203, 223) being positioned beyond the distal end (22) of the extremity-supporting base (20).

7. An apparatus as in claim 6, wherein said barrier shroud includes a dam (204, 224) near said drainage hole (203, 223) that assists in directing fluid and tissue toward said drainage hole (203, 223) during tissue irrigation and/or debridement.

8. An apparatus as in any one of claims 1 to 7, further comprising a drainage tube (187, 205, 225) in fluid communication with said drainage passageway (180, 203, 223) for drainage of fluid and tissue into a receiving vessel.

9. An apparatus as in claim 8, further comprising an aspiration device for aspirating fluid and tissue through said drainage tube (187, 205, 225).

10. An apparatus as in any one of claims 1 to 9, wherein said barrier shroud (200, 220) comprises a flexible sheet material.

11. An apparatus as in claim 10, further comprising one or more braces (207) that cooperate with said flexible sheet material of said barrier shroud (200) to maintain said barrier shroud (200) in a desired elevated configuration above said extremity-supporting base (20) and relative to an extremity during tissue irrigation and/or debridement.

12. An apparatus as in claim 10, further comprising one or more fasteners (228) that permit selective opening and closing of said barrier shroud (220) relative to an extremity placed on said extremity-supporting base (20).

13. An apparatus as in any one of claims 1 to 12, wherein said extremity-supporting base (10, 20) comprises an open-cell foam material, and optionally a flexible, fluid-impermeable coating over said open-cell foam material.

14. A method of capturing fluid and tissue produced while irrigating and/or debriding tissue of an extremity, comprising:
providing an apparatus as in any one of claims 1 to 13; collecting fluid and/or tissue resulting from tissue irrigation and/or debridement; and
the base (20) and barrier shroud (200, 220) substantially containing fluid and/or tissue resulting from tissue irrigation and/or debridement and controlling drainage of said fluid and/or tissue.

15. A method as in claim 14, further comprising aspirating fluid and/or tissue through a drainage tube (205, 225) in fluid communication with said barrier shroud (200, 220) and said base (20).

## Patentansprüche

1. Vorrichtung zum Abstützen einer Extremität während Gewebespülung und/oder Wundausschneidung und zum Auffangen von Fluid und Gewebe, die daraus resultieren, wobei sie umfasst:
eine Extremitäten stützende Basis (10, 20), die dazu ausgelegt ist, eine Extremität eines Patienten während Gewebespülung und/oder Wundausschneidung abzustützen, wobei die Basis (10, 20) ein proximales Ende (11, 21, 110), das während des Gebrauchs am nächsten zum Körper eines Patienten positioniert ist, und ein distales Ende (12, 22, 120) gegenüber dem proximalen Ende (11, 21, 110) aufweist, wobei die Basis (10, 20) dazu ausgelegt ist zu ermöglichen, dass sich ein Teil einer Extremität über das distale Ende (11, 21, 110) hinaus erstreckt,
wobei die Extremitäten stützende Basis (10, 20) eine Rinne (14, 24, 140) bildet, um eine Extremität auf der Extremitäten stützenden Basis (10, 20) zu halten, wobei sich die Rinne (14, 24, 140) entlang der Länge der Extremitäten stützenden Basis (10, 20) von einem Punkt am oder nahe am proximalen Ende (11, 21, 110) der Extremitäten stützenden Basis (10, 20) bis zu einem Punkt am oder nahe am distalen Ende (12, 22, 120) der Extremitäten stützenden Basis (10, 20) erstreckt, wobei die Rinne (14, 24, 140) zumindest zum proximalen Ende (11, 21, 110) der Extremitäten stützenden Basis (10, 20) hin ungeschlossen ist;
eine Barrierenumhüllung (200, 220), die derart positionierbar ist, so dass sie zumindest teilweise eine auf der Extremitäten stützenden Basis (10, 20) angeordnete Extremität einschließen kann; und
einen Ableitungskorridor (18, 180, 203, 223) in Fluidkommunikation mit der Extremitäten stützenden Basis (10, 20) und der Barrierenumhüllung (200, 220), der eine kontrollierte Ableitung von Fluid und Gewebe von der Extremitäten stützenden Basis (10, 20) und der Barrierenumhüllung (200, 220) während Gewebespülung und/oder Wundausschneidung bereitstellt.

2. Vorrichtung wie in Anspruch 1, wobei die Rinne (14, 24, 140) einen Rinnenboden (15, 25) und eine Seitenwand an jeder Seite des Rinnenbodens (15, 25) umfasst.

3. Vorrichtung wie in Anspruch 1 oder 2, wobei die Extremitäten stützende Basis (10, 20) nach unten zum Ableitungskorridor (18, 180, 203, 223) hin abgewinkelt ist, um die Bewegung von Fluid und Gewebe hin zum Ableitungskorridor (18, 180, 203, 223) während Gewebespülung und/oder Wundausschneidung zu erleichtern.

4. Vorrichtung wie in Anspruch 3, wobei der Ableitungskorridor (18, 180, 203, 223) am oder nahe am distalen Ende (12, 22, 120) der Extremitäten stützenden Basis (10, 20) positioniert ist, um die Bewegung von Fluid und Gewebe weg vom Körper des Patienten während Gewebespülung und/oder Wundausschneidung zu erleichtern.

5. Vorrichtung wie in Anspruch 3 oder 4, wobei der Ableitungskorridor ein Ableitungsloch (18, 180) durch die Extremitäten stützende Basis (10) an oder nahe an einem Tiefpunkt der nach unten abgewinkelten Basis (10) umfasst.

6. Vorrichtung wie in Anspruch 3 oder 4, wobei die Extremitäten stützende Basis (20) dazu ausgelegt ist, Fluide und/oder Gewebe hin zum distalen Ende (22) der Extremitäten stützenden Basis (20) oder darüber hinaus zu kanalisieren, wobei der Ableitungskorridor ein Ableitungsloch (203, 223) aufweist, das in der Barrierenumhüllung (200, 220) gebildet ist, wobei das Ableitungsloch (203, 223) über das distale Ende (22) der Extremitäten stützenden Basis (20) hinaus positioniert ist.

7. Vorrichtung wie in Anspruch 6, wobei die Barrierenumhüllung einen Damm (204, 224) nahe dem Ableitungsloch (203, 223) umfasst, der dabei hilft, Fluid und Gewebe hin zum Ableitungsloch (203, 223) während Gewebespülung und/oder Wundausschneidung zu lenken.

8. Vorrichtung wie in einem der Ansprüche 1 bis 7, die ferner ein Ableitungsrohr (187, 205, 225) in Fluidkommunikation mit dem Ableitungskorridor (180, 203, 223) zum Ableiten von Fluid und Gewebe in ein Aufhahmegefäß umfasst.

9. Vorrichtung wie in Anspruch 8, die ferner eine Ansaugvorrichtung zum Ansaugen von Fluid und Gewebe durch das Ableitungsrohr (187, 205, 225) umfasst.

10. Vorrichtung wie in einem der Ansprüche 1 bis 9, wobei die Barrierenumhüllung (200, 220) ein flexibles Lagenmaterial umfasst.

11. Vorrichtung wie in Anspruch 10, die ferner eine oder mehrere Klammern (207) umfasst, die mit dem flexiblen Lagenmaterial der Barrierenumhüllung (200) zusammenwirken, um die Barrierenumhüllung (200) in einer erwünschten erhöhten Konfiguration über der Extremitäten stützenden Basis (20) und in Bezug auf eine Extremität während Gewebespülung und/oder Wundausschneidung zu halten.

12. Vorrichtung wie in Anspruch 10, die ferner eine oder mehrere Befestigungen (228) umfasst, die ein selektives Öffnen und Schließen der Barrierenumhüllung (220) in Bezug auf eine auf der Extremitäten stützenden Basis (20) angeordnete Extremität zulassen.

13. Vorrichtung wie in einem der Ansprüche 1 bis 12, wobei die Extremitäten stützende Basis (10, 20) ein offenzelliges Schaummaterial und optional eine flexible, für Fluid undurchlässige Beschichtung über dem offenzelligen Schaummaterial umfasst.

14. Verfahren zum Auffangen von Fluid und Gewebe, das während der Spülung und/oder Wundausschneidung von Gewebe einer Extremität produziert wird, wobei dieses umfasst:
Bereitstellen einer Vorrichtung wie in einem der Ansprüche 1 bis 13;
Sammeln von Fluid und/oder Gewebe, das aus Gewebespülung und/oder Wundausschneidung resultiert;
und wobei
die Basis (20) und die Barrierenumhüllung (200, 220) im Wesentlichen Fluid und/oder Gewebe, das aus Gewebespülung und/oder Wundausschneidung resultiert, enthalten und die Ableitung dieses Fluids und/oder Gewebes kontrollieren.

15. Verfahren wie in Anspruch 14, das ferner das Ansaugen von Fluid und/oder Gewebe durch ein Ableitungsrohr (205, 225) in Fluidkommunikation mit der Barrierenumhüllung (200, 220) und der Basis (20) umfasst.

## Revendications

1. Appareil destiné à supporter une extrémité pendant un lavage et/ou un débridement de tissu et à capturer du fluide et du tissu ainsi obtenus, comprenant :
une base (10, 20) de support d'extrémité conçue pour supporter une extrémité d'un patient pendant un lavage et/ou un débridement de tissu, ladite base (10, 20) comportant une extrémité proximale (11, 21, 110) disposée au plus près du corps d'un patient pendant l'utilisation et une extrémité distale (12, 22, 120) située à l'opposé de ladite extrémité proximale (11, 21, 110), la base (10, 20) étant conçue pour permettre à une partie d'une extrémité de s'étendre au-delà de l'extrémité distale (11, 21, 110), dans lequel ladite base (10, 20) de support d'extrémité forme une gouttière (14, 24, 140) destinée à supporter une extrémité sur ladite base (10, 20) de support d'extrémité, ladite gouttière (14, 24, 140) s'étendant dans le sens de la longueur de la base (10, 20) de support d'extrémité d'un point se trouvant au niveau de l'extrémité proximale (11, 21, 110) de la base (10, 20) de support d'extrémité, ou à proximité de cette dernière, à un point se trouvant au niveau de l'extrémité distale (12, 22, 120) de la base (10, 20) de support d'extrémité, ou à proximité de cette dernière, ladite gouttière (14, 24, 140) n'étant pas fermée au moins en direction de l'extrémité proximale (11, 21, 110) de la base (10, 20) de support d'extrémité ;
une enveloppe protectrice (200, 220) qui peut être positionnée de façon à enfermer au moins partiellement une extrémité placée sur ladite base (10, 20) de support d'extrémité ; et
un conduit de drainage (18, 180, 203, 223) en communication fluidique avec ladite base (10, 20) de support d'extrémité et avec ladite enveloppe protectrice (200, 220) qui établit un drainage régulé de fluide et de tissu à partir de ladite base (10, 20) de support d'extrémité et de ladite enveloppe protectrice (200, 220) pendant un lavage et/ou un débridement de tissu.

2. Appareil selon la revendication 1, dans lequel ladite gouttière (14, 24, 140) comprend un fond (15, 25) de gouttière et une paroi latérale de chaque côté dudit fond (15, 25) de gouttière.

3. Appareil selon la revendication 1 ou la revendication 2, dans lequel ladite base (10, 20) de support d'extrémité est inclinée vers le bas en direction dudit conduit de drainage (18, 180, 203, 223) pour faciliter un déplacement de fluide et de tissu en direction dudit conduit de drainage (18, 180, 203, 223) pendant un lavage et/ou un débridement de tissu.

4. Appareil selon la revendication 3, dans lequel ledit conduit de drainage (18, 180, 203, 223) est disposé au niveau de ladite extrémité distale (12, 22, 120) de ladite base (10, 20) de support d'extrémité, ou à proximité de cette dernière, pour faciliter un déplacement de fluide et de tissu à l'écart du corps du patient pendant un lavage et/ou un débridement de tissu.

5. Appareil selon la revendication 3 ou la revendication 4, dans lequel ledit conduit de drainage comprend un trou de drainage (18, 180) ménagé à travers ladite base (10) de support d'extrémité au niveau d'un point bas, ou à proximité de ce dernier, de ladite base inclinée vers le bas (10).

6. Appareil selon la revendication 3 ou la revendication 4, dans lequel ladite base (20) de support d'extrémité est conçue pour canaliser des fluides et/ou du tissu en direction et au-delà de l'extrémité distale (22) de la base (20) de support d'extrémité, dans lequel ledit conduit de drainage comprend un trou de drainage (203, 223) formé dans ladite enveloppe protectrice (200, 220), le trou de drainage (203, 223) étant positionné au-delà de l'extrémité distale (22) de la base (20) de support d'extrémité.

7. Appareil selon la revendication 6, dans lequel ladite enveloppe protectrice comprend une digue (204, 224) à proximité dudit trou de drainage (203, 223) qui aide à diriger du fluide et du tissu en direction dudit trou de drainage (203, 223) pendant un lavage et/ou un débridement de tissu.

8. Appareil selon l'une quelconque des revendications 1 à 7, comprenant en outre un tube de drainage (187, 205, 225) en communication fluidique avec ledit conduit de drainage (180, 203, 223) à des fins de drainage de fluide et de tissu dans un récipient de réception.

9. Appareil selon la revendication 8, comprenant en outre un dispositif d'aspiration destiné à aspirer du fluide et du tissu à travers ledit tube de drainage (187, 205, 225).

10. Appareil selon l'une quelconque des revendications 1 à 9, dans lequel ladite enveloppe protectrice (200, 220) comprend un matériau en feuille souple.

11. Appareil selon la revendication 10, comprenant en outre un ou plusieurs arceaux (207) qui coopèrent avec ledit matériau en feuille souple de ladite enveloppe protectrice (200) pour maintenir ladite enveloppe protectrice (200) dans une configuration élevée souhaitée au-dessus de ladite base (20) de support d'extrémité et par rapport à une extrémité pendant un lavage et/ou un débridement de tissu.

12. Appareil selon la revendication 10, comprenant en outre une ou plusieurs attaches (228) qui permettent d'ouvrir et de fermer sélectivement ladite enveloppe protectrice (220) par rapport à une extrémité placée sur ladite base (20) de support d'extrémité.

13. Appareil selon l'une quelconque des revendications 1 à 12, dans lequel ladite base (10, 20) de support d'extrémité comprend un matériau en mousse à cellules ouvertes, et éventuellement un revêtement souple, imperméable au fluide sur ledit matériau en mousse à cellules ouvertes.

14. Méthode de capture de fluide et de tissu produits pendant un lavage ou un débridement de tissu d'une extrémité, consistant à :
utiliser un appareil selon l'une quelconque des revendications 1 à 13 ;
collecter du fluide et/ou du tissu obtenus à partir d'un lavage et/ou d'un débridement de tissu ; et
la base (20) et l'enveloppe protectrice (200, 220) contenant sensiblement du fluide et/ou du tissu obtenus à partir d'un lavage et/ ou d'un débridement de tissu et régulant un drainage dudit fluide et/ou dudit tissu.

15. Méthode selon la revendication 14, consistant en outre à aspirer du fluide et/ou du tissu à travers un tube de drainage (205, 225) en communication fluidique avec ladite enveloppe protectrice (200, 220) et avec ladite base (20).
